# EUROPEAN PATENT SPECIFICATION

(11) **EP 2 114 524 B1**
(45) Date of publication and mention of the grant of the patent: **07.01.2015**
(21) Application number: 07709396.1
(22) Date of filing: 28.02.2007
(51) Int. Cl.: A61N 1/365, A61B 5/0215, A61B 5/11

(54) **AN IMPLANTABLE HEART ANALYSING SYSTEM**
IMPLANTIERBARES HERZANALYSESYSTEM
SYSTÈME D'ANALYSE D'UN COEUR IMPLANTABLE

(43) Date of publication of application: 11.11.2009
(73) Proprietor: St. Jude Medical AB, 175 84 Järfälla (SE)
(72) Inventor: HOLMSTRÖM, Nils, S-175 57 Järfälla (SE); BLOMQVIST, Andreas, SE-187 76 Täby (SE); LARSSON, Berit, S-182 35 Danderyd (SE); JÄRVERUD, Karin, S-169 71 Solna (SE); HEDBERG, Sven-Erik, S-196 32 Kungsängen (SE)
(74) Representative: Sjölander, Henrik
(86) International application number: PCT/SE2007/000193
(87) International publication number: WO 2008/105690

(56) References cited:
- EP-A2- 0 473 070
- WO-A1-00/64344
- WO-A1-98/06454
- WO-A1-2006/135294
- WO-A1-2006/135294
- WO-A2-2004/066814
- US-A- 5 003 982
- US-A- 5 549 652
- US-A1- 2004 059 220
- US-B1- 6 473 647

## Description

### BACKGROUND OF THE INVENTION

### 1. Field of the invention

The present invention relates to an implantable heart analysing device, for analysing or monitoring the heart condition, in particular for analysing or monitoring the tension in a myocardial wall.

The invention also concerns an implantable heart analysing system that includes such an implantable heart analysing device.

### 2. Description of the prior art

US-A-5 544 656 describes an ultrasonic sonomicrometer apparatus capable of identifying the myocardial muscle/blood interface and continuously tracking this interface throughout the cardiac cycle using a piezoelectric transducer, which is secured to the epicardium of a ventricle. The apparatus can measure absolute myocardial wall thickness.

WO 2004/066814 A2 describes a cardiac sensor system that includes implanted cardiac sensor assemblies and an external controller which receives information from the implanted sensors. The sensors permit direct measurement of a number of physiologic parameters. The external controller permits calculation of a variety of performance values based on the measured physiological parameters. The system can include a sensor for sensing myocardial elasticity or contractility. This sensor may include a probe which may be mechanically advanced so that it engages myocardial tissue. By applying a known force, and measuring the degree of tissue displacement caused by the force, the elasticity or contractility can be calculated. Conversely, the probe could be moved by a known distance with the amount of required force being tracked in order to calculate elasticity or contractility.

US 6 702 777 B2 describes an apparatus for intracardiac drug administration, including a catheter which is inserted into a chamber of the heart and brought into engagement with a site in the heart wall. The apparatus can include (see column 14, lines 33-59) an ultrasound transducer, which emits a beam of ultrasonic radiation and receives ultrasound waves reflected from the heart wall. The transducer can be used to measure and map the thickness of the heart wall. The transducer may also be used to produce an ultrasound image of the endocardial surface.

WO 2004/096350 A1 describes, in connection with one embodiment in the document (see in particular pages 21-22), a lead with a tip electrode positioned adjacent to the left free ventricular wall. R-wave durations are obtained. For substantially constant ventricular conduction conditions, R-wave duration variations are analysed to determine ventricular wall thickening of a degree that indicates onset of chronic heart failure.

WO 2006/135294 A1 describes an implantable heart monitoring device comprising a control circuit arranged to generate a vibration signal suitable to actuate a vibrator. The control circuit is also adapted to communicate with at least a first implantable vibration sensor and to receive a detection signal from said vibration sensor. The vibrator is located at a distance from the vibration sensor. The control circuit is arranged to derive information concerning the mechanical properties of the heart, such as the stiffness and/or the thickness of at least a part of the heart.

### SUMMARY OF THE INVENTION

For certain patients it is important to be able to monitor the status of the heart, for example concerning the blood pressure in a certain heart chamber, for example, in the left atrium. An increased blood pressure in the left atrium may be a sign that the heart condition has become worse. An increased blood pressure will also have an effect on the tension of the heart wall surrounding the heart chamber in question. It would therefore be advantageous to be able to detect early symptoms of for example an increased tension in a wall of the heart.

One object of the present invention is to provide an implantable heart analysing device with which it is possible to derive information concerning the tension of a heart wall. A further object is to provide such a device that is able to monitor changes in the tension of the heart wall. Still an object of the invention is to provide such a device with a relatively simple construction.

The above objects are achieved by an implantable heart analysing device comprising a housing and a control circuit located within said housing,
the control circuit being adapted to generate an output signal adapted to actuate an activator, which is adapted to be positioned in contact with an inner or an outer wall of the heart, such that the output signal, when the device is connected to said activator positioned in contact with said wall of the heart, is able to make said wall of the heart deflect or vibrate,
the control circuit also being adapted to communicate with a sensor, which is identical with said activator that is adapted to be positioned in contact with the same wall of the heart as the activator, wherein the control circuit is adapted to from said sensor receive a sensor signal, which, when the sensor is positioned in contact with said wall of the heart, represents the movement of said heart wall,
wherein the control circuit is arranged to be able to carry out a procedure that involves the generation of at least one said output signal and sensing, during a certain time interval, a corresponding sensor signal, and wherein the control circuit is set up to be able to derive, based on the shape of the sensor signal sensed in response to the generated output signal, information concerning the tension of said heart wall.

With the help of the sensor signal, it is thus possible to detect the movement of the heart wall in response to a deflection or vibration generated with the help of the activator. The sensor signal therefore contains information of how the heart wall in question moves. The movement of the heart wall depends, inter alia, on the tension of the heart wall. With the help of a device according to the present invention, it is therefore possible to obtain information about the tension of the heart wall in question and thereby also information about the mechanical properties of the heart wall.

An early indication of a dysfunction of the heart may be an increased blood pressure in a certain heart chamber, for example in the left atrium. This increased blood pressure will also have an effect on the tension in the heart wall. With the present invention it is therefore, for example, possible to early detect a sign of an increased blood pressure in a certain heart chamber. This problem can therefore be treated at an early stage if the detection has been done early with the help of the present invention. An early detection, and a treatment, of for example increased blood pressure in the left atrium may make it possible to avoid the development of a severe congestive heart failure in the patient.

It should be noted that from some of the prior art documents disclosed above it is known to transmit an ultrasound wave and to analyse, for example, the time it takes for this wave to reach the detector. The present invention is not at all concerned with this kind of analysis. Instead, the present invention concerns the sensing of the actual behaviour of a heart wall (how the heart wall moves or vibrates) in response to a triggered deflection or vibration of the heart wall. This behaviour is detected by sensing the mentioned sensor signal.

With an inner wall of the heart is in this document meant a wall in the interior of the heart, for example, the septum between the atria or the septum between the ventricles.

With an outer wall of the heart is in this document meant a wall of the heart that is not located in the interior of the heart. In order to cause an outer wall of the heart to deflect or vibrate, the activator can for example with advantage be positioned inside the pericardium on the epicardium, or possibly outside the pericardium. The activator could, according to a less preferred embodiment, also be positioned in the heart (in an atrium or a ventricle) in contact with an outer wall.

With the "shape" of the sensor signal is meant any information contained in the sensor signal, which information is related to the tension of the heart wall. This information can for example be the attenuation of the sensor signal.

According to an embodiment of the implantable heart analysing device according to the invention, the device comprises at least one memory connected to the control circuit, wherein the control circuit is arranged to derive the information concerning the tension by comparing the shape of the sensor signal with a shape that has previously been stored in the memory. In the memory it is thus possible to store some kind of "template" that represents a typical sensor signal. A certain deviation from this stored template may then be an indication of, for example, the fact that the tension in the heart wall in question has increased.

According to a further embodiment of the implantable heart analysing device, the control circuit is arranged to be able to operate in time cycles corresponding to heart cycles. It is well known for example in connection with heart stimulating devices that such devices operate in time cycles corresponding to heart cycles.

According to a further embodiment, the control circuit is arranged such that said procedure is to be carried out during a portion of said time cycle that corresponds to a late part of the diastolic portion of the heart cycle. The diastolic portion can either be an atrial diastole or a ventricular diastole. It has thus appeared to be advantageous to measure the tension of the hear wall during such portions of the heart cycle. However, the device can also be arranged to carry out the procedure in question during other portions of the heart cycle.

If the activator/sensor is attached to an atrial wall, the procedure can for example be carried out around time of the P-wave (for example later than 100 ms before the expected occurrence of the P-wave, but before 50ms after the P-wave). If the activator/sensor is attached to a ventricular wall, the procedure can for example be carried out around the time of the QRS-complex (for example later than 50 ms before the expected occurrence of the QRS-complex, but before 150ms after the QRS-complex).

According to a further embodiment, the control circuit is arranged to carry out said procedure at a plurality of occasions, and to store a result from said procedure in said memory, such that information can be derived of how said tension of the heart wall has changed between these occasions. It is thereby possible to monitor how the tension of the heart wall in question has changed over time.

According to a further embodiment, the control circuit is arranged such that said procedure is carried out at the same portion of said time cycle at the different occasions. In order to monitor how the tension of the heart wall changes over time, it is advantageous to carry out the procedure during the same portion of the heart cycle.

According to a further embodiment, the control circuit is arranged such that said procedure is carried out at a plurality of occasions within the same time cycle. By carrying out the procedure at different occasions within the same time cycle, it is possible to detect how the tension of the heart wall has changed during the same time cycle. It is, for example, possible to first carry out the procedure during the time of the QRS-complex and then at a second occasion after the contraction of the heart.

According to a further embodiment, the control circuit is arranged such that a relationship between at least two such sensor signals within the same time cycle is stored in a memory. It is hereby possible to obtain information of for example the ratio between the tensions at two different occasions within the same time cycle. Also such a relationship may provide information about the condition of the heart. For example, in a patient suffering from congestive heart failure it appears that the tension in the heart wall may remain to a certain extent also during the diastolic portion of the heart cycle.

According to a further embodiment, the control circuit is arranged such that said relationship is formed during different time cycles, such that information can be derived of how said relationship has changed between these different time cycles. It is hereby possible to see how the mentioned relationship changes over time. A certain change of the relationship (for example the mentioned ratio) may indicate that the heart condition has become worse.

According to a further embodiment, the control circuit is arranged such that the output signal is a short pulse or voltage step. It has appeared to be advantageous to actuate the activator with such a short pulse or voltage step.

According to a further embodiment, said activator is a piezoelectric device. With advantage, a piezoelectric device can be used as the activator.

According to a further embodiment, the control circuit is arranged such that said time interval begins within 30 ms after the generation of said output signal. It is advantageous to start the sensing very soon after the generation of the output signal, while the heart still moves or vibrates substantially in response to the activation caused by the activator.

According to a further embodiment, the control circuit is arranged such that said time interval is less than 200ms long. It has also appeared to be advantageous to only carry out the detection procedure during a short time interval.

The mentioned time interval, during which the sensor signal is sensed is with advantage at least 20ms long, preferably more than 30ms long. The time interval is however, with advantage less than 150 ms long, preferably less than 100ms long. The time interval can for example be 80ms long. Preferably, the time interval begins directly after that the output signal has been delivered.

According to a further embodiment, the control circuit is arranged such that the attenuation of the sensor signal during said time interval is used as a measure of the tension of the heart wall. It has appeared to be advantageous to use the attenuation of the sensor signal as an indication of the tension of the heart wall. However, also other features of the sensor signal may be used as an indication of the tension of the heart wall.

Another aspect of the present invention concerns an implantable heart analysing system. According to the invention, this system comprises an implantable heart analysing device according to any one of the preceding embodiments together with said activator and said sensor, which is identical with said activator, wherein the control circuit is set up to communicate with said activator and sensor. Such a system according to the invention has advantages corresponding to those described above in connection with the device.

A method of analysing the tension of a wall of a heart in a human or animal being is disclosed. The method comprises the following steps:
position an activator in contact with an inner or an outer wall of the heart,
position a sensor in contact with the same wall of the heart as the activator, wherein the sensor is identical with said activator,
carry out a procedure which involves the generation of at least one output signal that actuates the activator, such that the activator makes said wall of the heart deflect or vibrate, and sensing, during a certain time interval, a corresponding sensor signal from said sensor, which sensor signal represents the movement of said heart wall, and deriving, based on the shape of the sensor signal sensed in response to the generated output signal, information concerning the tension of said heart wall.

Different advantageous manners of carrying out the method are clear from the description below.

The method has similar advantages to those described above in connection with the device according to the invention.

Further aspects and advantages of the present invention will become clear from the following description and claims.

### BRIEF DESCRIPTION OF THE DRAWINGS

Fig 1 shows schematically an embodiment of an implantable heart analysing device according to the invention connected to leads with sensing and pacing electrodes and an activator and a sensor positioned in or at a heart.
Fig 2 shows schematically a control circuit which may form part of the device.
Fig 3 shows schematically a signal for actuating an activator as well as a subsequent sensor signal.
Fig 4 shows schematically the same kind of signal for actuating an activator as in Fig 3, but a different sensor signal, obtained as a different occasion than the one shown in Fig 3.
Fig 5 shows schematically a slightly different manner of actuating an activator than the one shown in Fig 3 and 4.
Fig 6 illustrates schematically a method.
Fig 7 illustrates schematically another manner of carrying out the method.

### DESCRIPTION OF PREFERRED EMBODIMENTS

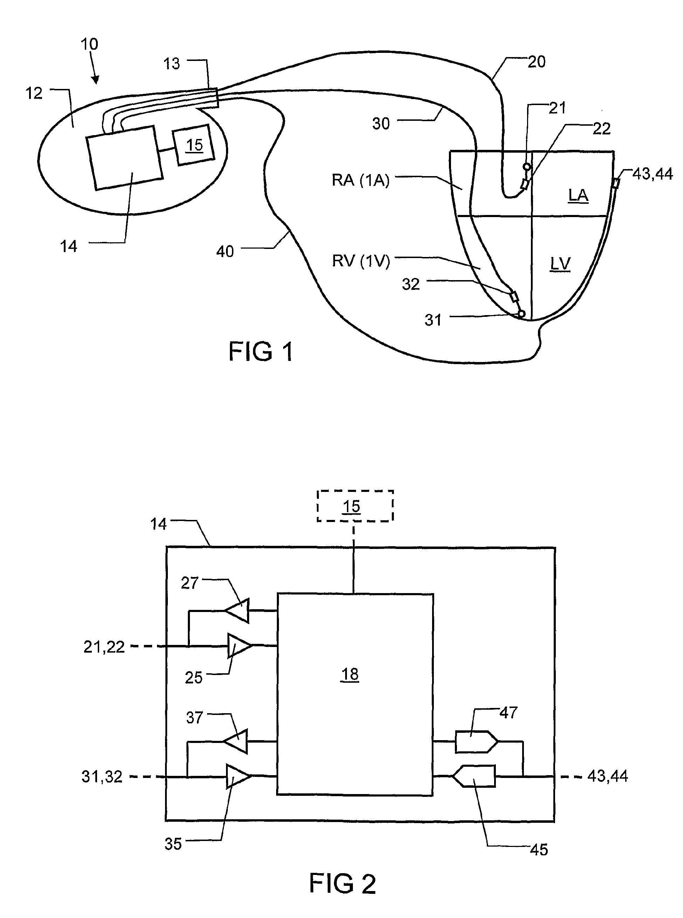

Fig 1 shows schematically an implantable heart analysing device 10 according to the invention. According to the shown embodiment, the device 10 also functions as a heart stimulating device. The device 10 comprises a housing 12. The device 10 has a connector portion 13. Via the connector portion 13, the device 10 can be connected to different leads. In Fig 1 the device 10 is connected to three leads 20, 30 and 40.

Fig 1 also schematically illustrates a heart with a right atrium RA, a left atrium LA, a right ventricle RV and a left ventricle LV.

The lead 20 includes a pacing and sensing electrode 21, 22.

Similarly to the lead 20, the lead 30 includes a pacing and sensing electrode 31,32.

The lead 40 is connected to an activator 43 in the form of a piezoelectric device. According to this embodiment, the activator 43 also acts as a sensor 44 for sensing the movement of a heart wall. However, according to an alternative embodiment not part of the invention, the sensor 44 can be separate from the activator 43. If the sensor 44 is separate from the activator 43, the sensor 44 should preferably still be positioned close to the activator 43 at the same heart wall as the activator 43. In the shown embodiment, the activator/sensor 43, 44 is positioned on the heart wall outside the left atrium LA. The activator/sensor 43, 44 may for example be introduced into the pericardium and be positioned in the pericardium on the epicardium at the left atrium. However, it is also possible that the activator/sensor 43,44 is introduced into the heart and positioned for example at or in the septum between the left and right atria.

In the shown example, the electrodes 21, 22, 31, 32 are bipolar electrodes with a tip portion 21, 31 and a ring portion 22, 32. However, it is also possible to use unipolar electrodes. This is known to a person skilled in the art.

The device 10 comprises a control circuit 14, which will be described further below. The device 10 also comprises at least one memory 15 connected to the control circuit 14.

The device 10 together with the leads 20, 30, 40 and the pacing/sensing electrodes 21, 22, 31, 32 and the activator/sensor 43, 44 constitute a heart stimulating system according to the invention. This system can be implanted in a patient.

It can be noted that figure 1 shows a device 10 which can electrically pace and sense both an atrium and a ventricle. However, also other alternatives are possible. The device 10 can of course also constitute an analysing device 10, which is not designed to pace and sense the heart.

In Fig 1, the electrode 21, 22 constitutes a first atrial sensing and/or pacing electrode 21, 22 which is positioned in a first atrium 1A of the heart, according to this embodiment the right atrium RA, in order to enable sensing and/or pacing of this atrium RA.

The electrode 31, 32 constitutes a first ventricular sensing and pacing electrode 31, 32, which is positioned in a first ventricle 1 V of the heart, in this embodiment the right ventricle RV. The first ventricular sensing and pacing electrode 31, 32 is adapted to enable sensing and pacing of this first ventricle 1 V.

It is also possible that the device 10 is connected to further leads and/or further sensing/pacing electrodes, for example electrodes positioned in order to sense and/or pace the left ventricle LV and/or the left atrium LA and electrodes designed to enable defibrillation.

Fig 2 shows schematically the control circuit 14 in some more detail. The control circuit 14 includes a control portion 18 connected to a memory 15.

The control circuit 14 includes a first atrial sensing and/or pacing circuit 25, 27. In this embodiment, this circuit 25, 27 includes a sensing circuit 25 and a pacing circuit 27. The first atrial sensing and/or pacing circuit 25, 27 communicates with the first atrial sensing and/or pacing electrode 21, 22 via the lead 20. The first atrial sensing and/or pacing circuit 25, 27 is thus adapted to sense and/or pace a first atrium 1 A, in this case the right atrium RA.

The control circuit 14 also includes a first ventricular sensing circuit 35 and a first ventricular pacing circuit 37. These circuits 35, 37 communicate with the first ventricular sensing and pacing electrode 31, 32 via the lead 30. The circuits 35, 37 are thus adapted to sense and pace a first ventricle 1V, in this case the right ventricle RV.

Although not described in any detail here, the device 10 can be arranged to include several other operational features that are known in connection with heart stimulation/analysing devices. Such normal features include, for example that the control portion 18 is set up to operate with blanking and refractory periods; the ability to deliver back-up pulses if a heart chamber is not captured when a pacing pulse has been delivered; the ability to perform capture threshold searches; the ability to carry out defibrillation; an activity sensor for sensing the activity of the patient in whom the device has been implanted; the ability to sense impedance values; the ability to sense chemical substances in the blood; the ability to communicate with the help of so-called telemetry, etc.

The control circuit 14 is arranged to be able to operate in time cycles corresponding to heart cycles. Such an operation is normal for an implantable heart stimulating device. The time cycles are determined by preset timer intervals which also may depend on detected signals.

The control circuit 14 also comprises a circuit 47 adapted to generate an output signal in order to actuate the activator 43. Furthermore, the control circuit 14 comprises a circuit 45 adapted to receive a sensor signal from the sensor 44 and to thereby detect the movement of the heart wall on which the sensor 44 is positioned.

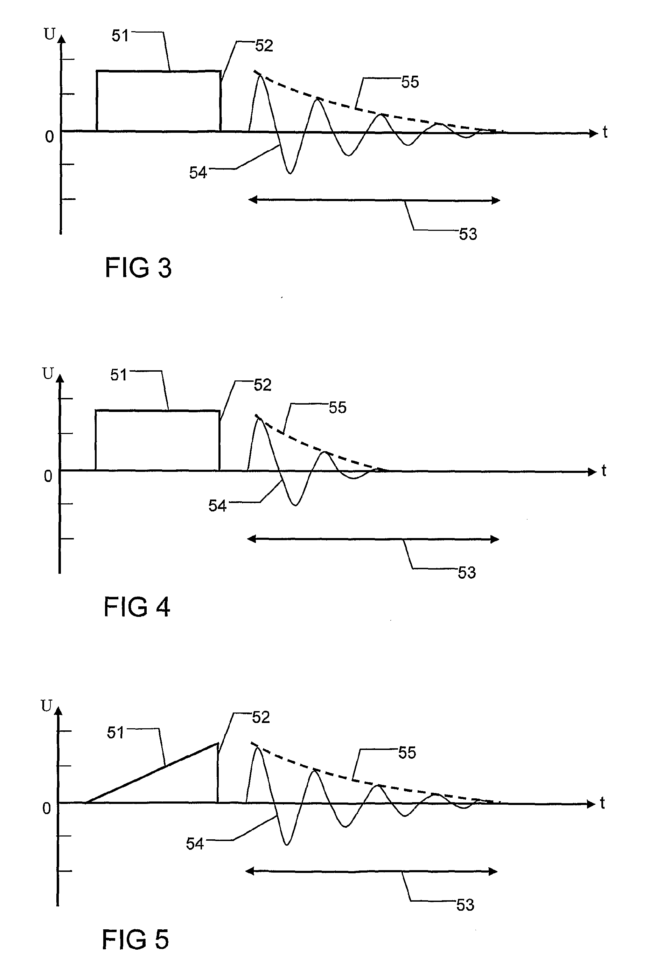

Fig 3 shows voltage U as a function of time t and illustrates schematically how an output signal to the activator 43 can be generated. The activator 43 can thus be a piezoelectric crystal or other piezoelectric device. The control circuit 14 is arranged to apply a voltage 51 over the activator 43. When the circuit to the activator 43 is short-circuited (shown at 52) or reversed the thickness (or the deflection) of the activator 43 changes rapidly. The voltage drop 52 thus constitutes an output signal to the activator 43 which causes the activator to make the wall of the heart deflect or vibrate.

During a certain time interval (sensing window) 53, the control circuit 14 senses a sensor signal 54 which represents the movement of the heart wall. The sensor signal 54 is received from the sensor 44, which may be the same device as the activator 43. The sensing window 53 may for example start 10 ms after the generation of the output signal 52. The sensing window 53 may for example be 80 ms long. The shape of the sensor signal 54 contains information concerning the tension of the heart wall. The attenuation of the sensor signal 54 is represented by 55. The attenuation 55 of the sensor signal is an indication of the tension of the heart wall.

In Fig 3 it is shown that the voltage of the sensor signal 54 has both positive and negative values. This is an indication of the fact that the heart wall vibrates. Also the frequency of this vibration is related to the tension of the heart wall. It should however be noted that it is not always the case that the heart wall will vibrate in response to an actuation caused by the activator 53. Instead it is possible that the heart wall regains its previous shape without vibration. However, even in this case, the time it takes for the heart wall to regain its shape is an indication of the tension. Information concerning the tension of the heart wall can also be obtained by comparing the shape of the sensor signal 54 with the shape of a template that has previously been stored in the memory 15.

The control circuit 14 is arranged or programmed to carry out the procedure illustrated in Fig 3 at different occasions. This procedure can, for example, when an atrial heart wall is sensed, be carried out around the time of the P-wave, or at a late part of the diastolic portion of the heart cycle.

The control circuit 14 can be arranged to carry out the procedure at a plurality of occasions, and to, at each occasion, store the result from the procedure in the memory 15. In this way, information can be derived of how the tension of the heart wall has changed between these occasions.

Fig 4 illustrates schematically the procedure corresponding to Fig 3 performed at a later occasion. As can be seen in Fig 4, the sensor signal 54 is more attenuated in Fig 4 than in Fig 3. This is an indication of the fact that the tension of the heart wall in question has changed.

Preferably, the control circuit 14 is arranged such that the procedure is carried out at the same portion of the time cycle at the different occasions.

It is also possible that the control circuit 14 is arranged such that the procedure is carried out at a plurality of occasions within the same time cycle. For example, the procedure can be carried out at two different occasions within the same heart cycle. The control circuit 14 can thereby be arranged such that a relationship between the two sensor signals from these two occasions is stored in the memory 15. The relationship may for example be the ratio between the attenuations 55.

The control circuit 14 can be arranged such that this relationship is formed during different time cycles. In this way it is possible to derive information of how the relationship has changed between the different time cycles.

Fig 5 illustrates schematically a slightly different manner of producing the output signal 52 than the manner shown in Fig 3. According to Fig 5, the voltage over the piezoelectric activator 43 is gradually increased. Then the circuit is short-circuited in order to provide the output signal 52.

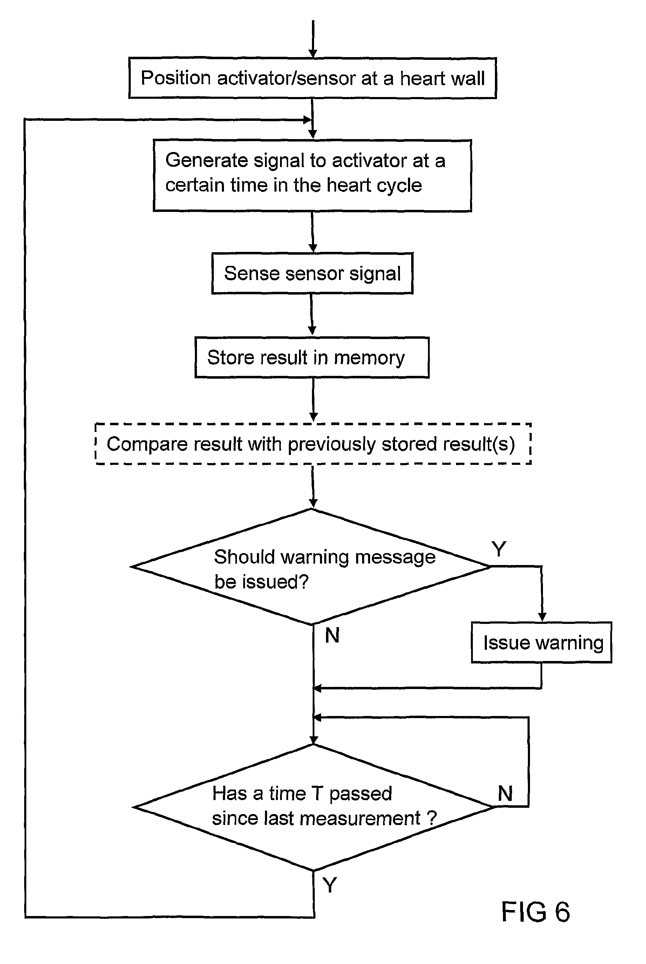

A manner of carrying out the method will now be described with reference to Fig 6.

An activator 43 is positioned in contact with an inner or an outer wall of the heart. The activator 43 can, for example, be positioned in the pericardium of the heart, for example in contact with the epicardium outside the left atrium LA. Alternatively, the activator 43 can be positioned in the heart, for example in the septum between the atria.

A sensor 44 is positioned in contact with the same wall of the heart as the activator 43. The sensor 44 can be identical with the activator 43. The activator 43 and the sensor 44 may be piezoelectric devices.

A procedure is carried out in order to obtain information concerning the tension of the heart wall. This procedure can, for example, be carried out during a late part of the diastolic portion of the heart cycle. If an atrial heart wall is to be sensed, the procedure can, for example, be performed about the time of the P-wave.

This procedure involves the generation of an output signal that actuates the activator 43. The output signal can be a short voltage step 52. The activator 43 will cause the wall of the heart to deflect or vibrate, preferably by producing a short impulse on the heart wall. A corresponding sensor signal 54 from the sensor 44 is then sensed during a certain time interval 53. The time interval 53 can, for example, begin 10 ms after the generation of the output signal 52. The time interval 53 can, for example, be 80ms long. The sensor signal 54 represents the movement of the heart wall. Based on the shape of the sensor signal 54 sensed in response to the generated output signal 52, in- formation concerning the tension of the heart wall can be derived. This can for example be done by comparing the shape of the sensor signal 54 with a shape that has previously been stored in a memory 15. For example the attenuation of the sensor signal 54 during the time interval 53 can be used as a measure of the tension of the heart wall. The result of the procedure is stored in the memory 15.

The method may also include the generation of a warning message, for example if the tension of the heart wall is higher than a predetermined value or if the tension has changed more than a predetermined amount. The warning message can for example be that a message is stored in the memory 15, such that this message can later be retrieved by the physician. The warning message may also be provided to a health care provider via Radio Frequency Telemetry to e.g. a home monitoring unit that relays the warning information to the health care provider. Alternatively, the warning, for example in the form of a vibration of the hosing 12, could alert the patient.

The method so far described can be carried out again if a certain time has passed. The procedure can thus be carried out at a plurality of occasions, for example once a day. Preferably, the procedure is carried out at the same portion of the heart cycle at the different occasions. At each occasion, the result from said procedure is stored in a memory 15. Hereby, it is possible to derive information of how the tension of the heart wall has changed between these occasions. It should be noted that at each "occasion" the procedure can in fact be carried out a plurality of times, in order to obtain a statistically more correct value of the tension of the heart wall.

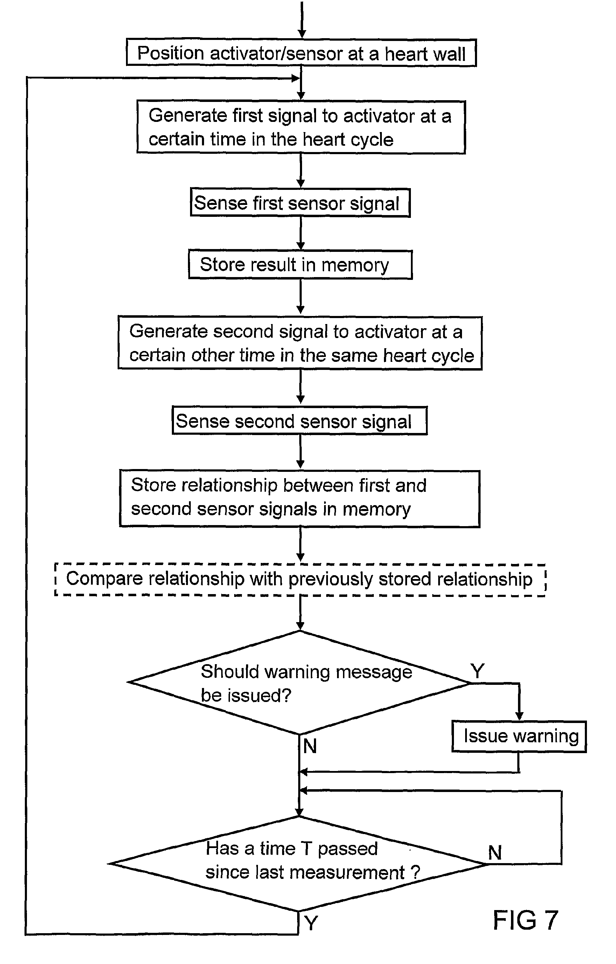

Fig 7 illustrates another manner of carrying out the method. This manner is basically the same as the one illustrated in Fig 6. However, the method of Fig 7 differs from the method of Fig 6 in that the mentioned procedure is carried out twice within the same heart cycle, for example once about the time of the P-wave and once after the contraction of the heart. It is of course within the scope of the invention that the procedure is carried out more than two times during the same heart cycle.

The result from each procedure is stored in the memory 15. According to one possible manner of carrying out the method, a relationship between at least two such sensor signals 54 within the same heart cycle is stored in the memory 15. This relationship can for example be the ratio between the attenuations at the two different procedures.

Similarly to the method of Fig 6, a warning message can be issued, for example if the mentioned relationship fulfils a predetermined criteria.

Also analogously to the method illustrated in Fig 6, the whole method described so far can be carried out at regular intervals, for example once a day. The relationship can thus be formed during different heart cycles, such that information can be derived of how said relationship has changed between these different heart cycles. The warning message can thus for example be issued if the mentioned relationship has changed more than a predetermined amount between the different times when the method is carried out.

The invention is not limited to the described embodiments but may be varied and modified within the scope of the following claims.

## Claims

1. An implantable heart analysing system, comprising:
an activator (43) adapted to make the wall of the heart deflect or vibrate;
a sensor (44) adapted to generate a sensor signal which represents movement of the heart wall; a lead (40) connected to said activator (43) and to said sensor (44);
an implantable heart analysing device (10) comprising:
a housing (12);
a connector portion (13) adapted to connect said lead (40);
a control circuit (14) located within said housing (12) and connected to said connector portion (13) to communicate with said activator (43) and said sensor (44);
at least one memory (15) connected to the control circuit (14),
said control circuit (14) is adapted to generate an output signal adapted to actuate said activator (43) and to receive said sensor signal from said sensor (44);
said control circuit (14) is arranged to be able to carry out a procedure that involves generation of said output signal and sensing, during a certain time interval, said sensor signal;
said control circuit (14) is adapted to derive information concerning tension of a heart wall of a heart based on i) attenuation of the sensor signal during said time interval, or ii) a comparison of a shape of said sensor signal with a shape of a template that has previously been stored in said at least one memory (15), **characterized in that**
said activator (43) is adapted to be positioned in contact with an inner or an outer heart wall of said heart; and
said sensor (44) is identical with said activator (43).

2. An implantable heart analysing system according to claim 1, wherein said control circuit (14) is arranged to be able to operate in time cycles corresponding to heart cycles.

3. An implantable heart analysing system according to claim 2, wherein said control circuit (14) is arranged such that said procedure is to be carried out during a portion of said time cycle that corresponds to a late part of the diastolic portion of the heart cycle.

4. An implantable heart analysing system according to any of the preceding claims, wherein said control circuit (14) is arranged to carry out said procedure at a plurality of occasions, and to store a result from said procedure in said at least one memory (15), such that information can be derived of how said tension of said heart wall has changed between said plurality of occasions.

5. An implantable heart analysing system according to claim 4, wherein said control circuit (14) is arranged such that said procedure is carried out at the same portion of said time cycle at said plurality of occasions.

6. An implantable heart analysing system according to claim 2, optionally in combination with any further one of the preceding claims, wherein said control circuit (14) is arranged such that said procedure is carried out at a plurality of occasions within the same time cycle.

7. An implantable heart analysing system according to claim 6, wherein said control circuit (14) is arranged such that a relationship between at least two such sensor signals within the same time cycle is stored in said at least one memory (15).

8. An implantable heart analysing system according to claim 7, wherein said control circuit (14) is arranged such that said relationship is formed during different time cycles, such that information can be derived of how said relationship has changed between these different time cycles.

9. An implantable heart analysing system according to any one of the preceding claims, wherein said control circuit (14) is arranged such that said output signal is a short pulse or voltage step.

10. An implantable heart analysing system according to any one of the preceding claims, wherein said activator (43) is a piezoelectric device.

11. An implantable heart analysing system according to any one of the preceding claims, wherein said control circuit (14) is arranged such that said time interval begins within 30 ms after generation of said output signal.

12. An implantable heart analysing system according to any one of the preceding claims, wherein said control circuit (14) is arranged such that said time interval is less than 200 ms long.

## Patentansprüche

1. Implantierbares Herzanalysesystem, umfassend:
einen Aktivator (43), der geeignet ist zur Biegung der Wand des Herzens oder um diese in Schwingungen zu versetzen;
einen Sensor (44), der geeignet ist zur Erzeugung eines Sensorsignals, das eine Bewegung der Herzwand darstellt;
eine Leitung (40), die mit dem Aktivator (43) und dem Sensor (44) verbunden ist;
eine implantierbare Herzanalysevorrichtung (10), umfassend:
ein Gehäuse (12);
eine Steckverbindung (13), die geeignet ist zum Anschluss der Leitung (40);
einen Steuerkreis (14), der in dem Gehäuse (12) angeordnet ist und an die Steckverbindung (13) angeschlossen ist, um mit dem Aktivator (43) und dem Sensor (44) zu kommunizieren;
wenigstens einen Speicher (15), der an den Steuerkreis (14) angeschlossen ist, wobei
der Steuerkreis (14) geeignet ist zur Erzeugung eines Ausgangssignals zur Betätigung des Aktivators (43) und zum Empfang des Sensorsignals von dem Sensor (44);
der Steuerkreis (14) derart angeordnet ist, um ein Verfahren auszuführen, das die Erzeugung des Ausgangssignals und eine Messung des Sensorsignals während eines bestimmten Zeitintervalls beinhaltet;
der Steuerkreis (14) geeignet ist, Informationen über die Anspannung einer Herzwand eines Herzens abzuleiten, basierend auf i) einer Dämpfung des Sensorsignals während des Zeitintervalls, oder ii) einem Vergleich einer Form des Sensorsignals mit einer Form einer Vorlage, die zuvor in dem wenigstens einen Speicher (15) gespeichert worden ist, **dadurch gekennzeichnet, dass**
der Aktivator geeignet ist, in Kontakt mit einer inneren oder einer äußeren Herzwand des Herzens positioniert zu werden; und
der Sensor (44) mit dem Aktivator (43) identisch ist.

2. Implantierbares Herzanalysesystem nach Anspruch 1, bei dem der Steuerkreis (14) angepasst ist, um in Zeitzyklen entsprechend den Herzzyklen betrieben zu werden.

3. Implantierbares Herzanalysesystem nach Anspruch 2, bei dem der Steuerkreis (14) derart angeordnet ist, dass das Verfahren während eines Abschnitts des Zeitzyklus ausgeführt werden soll, der mit einem späten Teil des diastolischen Abschnitts des Herzzyklus übereinstimmt.

4. Implantierbares Herzanalysesystem nach einem der vorhergehenden Ansprüche, bei dem der Steuerkreis (14) derart angeordnet ist, das Verfahren bei einer Vielzahl von Ereignissen auszuführen und ein Ergebnis des Verfahrens in dem wenigstens einen Speicher (15) zu speichern, derart, dass Informationen abgeleitet werden können, wie sich die Anspannung der Herzwand zwischen der Vielzahl der Ereignisse verändert hat.

5. Implantierbares Herzanalysesystem nach Anspruch 4, bei dem der Steuerkreis (14) so angeordnet ist, dass das Verfahren während desselben Abschnitts des Zeitzyklus bei der Vielzahl von Ereignissen ausgeführt wird.

6. Implantierbares Herzanalysesystem nach Anspruch 2, wahlweise in Kombination mit einem weiteren der vorhergehenden Ansprüche, bei dem der Steuerkreis (14) so angeordnet ist, dass das Verfahren bei einer Vielzahl von Ereignissen innerhalb desselben Zeitzyklus ausgeführt wird.

7. Implantierbares Herzanalysesystem nach Anspruch 6, bei dem der Steuerkreis (14) so angeordnet ist, dass ein Verhältnis zwischen wenigstens zwei solcher Sensorsignale innerhalb desselben Zeitzyklus in dem wenigstens einen Speicher (15) gespeichert wird.

8. Implantierbares Herzanalysesystem nach Anspruch 7, bei dem der Steuerkreis (14) so angeordnet ist, dass das Verhältnis während unterschiedlicher Zeitzyklen gebildet wird, so dass Informationen abgeleitet werden können, wie sich das Verhältnis zwischen diesen unterschiedlichen Zeitzyklen verändert hat.

9. Implantierbares Herzanalysesystem nach einem der vorhergehenden Ansprüche, bei dem der Steuerkreis (14) so angeordnet ist, dass das Ausgangssignal ein kurzer Impuls oder eine Spannungsstufe ist.

10. Implantierbares Herzanalysesystem nach einem der vorhergehenden Ansprüche, bei dem der Aktivator (43) eine piezoelektrische Vorrichtung ist.

11. Implantierbares Herzanalysesystem nach einem der vorhergehenden Ansprüche, bei dem der Steuerkreis (14) so angeordnet ist, dass das Zeitintervall innerhalb 30 ms nach einer Erzeugung des Ausgangssignals beginnt.

12. Implantierbares Herzanalysesystem nach einem der vorhergehenden Ansprüche, bei dem der Steuerkreis (14) so angeordnet ist, dass das Zeitintervall weniger als 200 ms lang ist.

## Revendications

1. Système d'analyse cardiaque implantable, comprenant :
un activateur (43) adapté pour faire se déformer ou vibrer la paroi du coeur ;
un capteur (44) adapté pour générer un signal de capteur qui représente le mouvement de la paroi cardiaque ;
un conducteur (40) raccordé audit activateur (43) et audit capteur (44) ;
un dispositif d'analyse cardiaque implantable (10) comprenant :
un logement (12) ;
une partie formant connecteur (13) adaptée pour raccorder ledit conducteur (40) ;
un circuit de commande (14) situé à l'intérieur dudit logement (12) et raccordé à ladite partie formant connecteur (13) pour communiquer avec ledit activateur (43) et ledit capteur (44) ;
au moins une mémoire (15) raccordée au circuit de commande (14), ledit circuit de commande (14) étant adapté pour générer un signal de sortie adapté pour actionner ledit activateur (43) et pour recevoir ledit signal de capteur en provenance dudit capteur (44) ;
ledit circuit de commande (14) étant agencé pour pouvoir réaliser une procédure impliquant la génération dudit signal de sortie et la détection, pendant un certain intervalle de temps, dudit signal de capteur ;
ledit circuit de commande (14) étant adapté pour obtenir des informations concernant la tension d'une paroi d'un coeur en se fondant sur i) l'atténuation du signal de capteur pendant ledit intervalle de temps, ou ii) une comparaison d'une forme dudit signal de capteur avec une forme d'un modèle précédemment stocké dans ladite au moins une mémoire (15),
**caractérisé en ce que :**
ledit activateur (43) est adapté pour être positionné en contact avec une paroi intérieure ou extérieure dudit coeur ; et
ledit capteur (44) est identique audit activateur (43).

2. Système d'analyse cardiaque implantable selon la revendication 1, dans lequel ledit circuit de commande (14) est agencé pour pouvoir fonctionner selon des cycles temporels correspondant aux cycles cardiaques.

3. Système d'analyse cardiaque implantable selon la revendication 2, dans lequel ledit circuit de commande (14) est agencé de telle manière que ladite procédure soit réalisée pendant une partie dudit cycle temporel correspondant à une partie tardive de la partie diastolique du cycle cardiaque.

4. Système d'analyse cardiaque implantable selon l'une quelconque des revendications précédentes, dans lequel ledit circuit de commande (14) est agencé pour réaliser ladite procédure dans une pluralité d'occasions, et pour stocker un résultat de ladite procédure dans ladite au moins une mémoire (15), de telle manière que des informations puissent être obtenues sur la façon dont ladite tension de ladite paroi cardiaque a changé entre ladite pluralité d'occasions.

5. Système d'analyse cardiaque implantable selon la revendication 4, dans lequel ledit circuit de commande (14) est agencé de telle manière que ladite procédure soit réalisée à la même partie dudit cycle temporel à ladite pluralité d'occasions.

6. Système d'analyse cardiaque implantable selon la revendication 2, éventuellement en combinaison avec une autre des revendications précédentes, dans lequel ledit circuit de commande (14) est agencé de telle manière que ladite procédure soit réalisée en une pluralité d'occasions au cours du même cycle temporel.

7. Système d'analyse cardiaque implantable selon la revendication 6, dans lequel ledit circuit de commande (14) est agencé de telle manière qu'une relation entre au moins deux signaux de capteur dans le même cycle temporel soit stockée dans ladite au moins une mémoire (15).

8. Système d'analyse cardiaque implantable selon la revendication 7, dans lequel ledit circuit de commande (14) est agencé de telle manière que ladite relation soit formée pendant différents cycles temporels, de telle manière que des informations puissent être obtenues sur la façon dont ladite relation a changé entre ces différents cycles temporels.

9. Système d'analyse cardiaque implantable selon l'une quelconque des revendications précédentes, dans lequel ledit circuit de commande (14) est agencé de telle manière que ledit signal de sortie soit une impulsion brève ou un pas de tension bref.

10. Système d'analyse cardiaque implantable selon l'une quelconque des revendications précédentes, dans lequel ledit activateur (43) est un dispositif piézoélectrique.

11. Système d'analyse cardiaque implantable selon l'une quelconque des revendications précédentes, dans lequel ledit circuit de commande (14) est agencé de telle manière que ledit intervalle de temps commence dans un délai de 30 ms après la génération dudit signal de sortie.

12. Système d'analyse cardiaque implantable selon l'une quelconque des revendications précédentes, dans lequel ledit circuit de commande (14) est agencé de telle manière que ledit intervalle de temps soit de moins de 200 ms.
